# EUROPEAN PATENT APPLICATION

(11) **EP 1 745 766 A2**
(43) Date of publication of application: **24.01.2007**
(21) Application number: 06017099.0
(22) Date of filing: 06.04.1998
(51) Int. Cl.: A61F 13/15

(54) **Disposable garments and their manufacturing**

(30) Priority: 08.04.1997 US 841988; 08.04.1997 US 841961
(62) Divisional of application: 98914522.2
(71) Applicant: KIMBERLY-CLARK WORLDWIDE, INC., Neenah, Wisconsin 54956 (US)
(72) Inventor: Alberts, Joseph, Richard, Menasha, WI 54952 (US); Drezdzon, Edward, Anthony, II, Appleton, WI 54914 (US); Fries, Donald, Merlin, Combined Locks, WI 54952 (US); Konetzke, Richard, Mark, Menesha, WI 54952 (US); Kvitek, Thomas, Theodore, Menasha, WI 54952 (US); Muhlebach, Michel, Joseph, Menasha, WI 54952 (US); Nelson, Michael, Joseph, Neenah, WI 54956 (US); Rabe, Gerald, Leigh, Appleton, WI 54915 (US); Ribble, Brendon, Frank, Menesha, WI 54952 (US); Roth, James, Frederik, Appleton, WI 54915 (US); Wittman, Jon, Mark, Combined Locks, WI 54113 (US); Rosch, Paulette, Mary, Sherwood, WI 54169 (US)
(74) Representative: Booth, Catherine Louise

(57) **Abstract**

A disposable garment is provided. The disposable garment comprises a pant structure having a front waist band region and a back waist band region longitudinally spaced and terminating in longitudinal ends, a crotch area between the front and back waist band regions, and a pair of side panels wherein the side panels of the pant structure include elastic elements, the pant structure having a waist opening, two leg openings and waist elastic members. The disposable garment may further comprise a skirt cover or a trunk cover. The skirt cover or trunk cover has at least a front panel and a back panel, each having waist regions. The waist region of the front panel and the waist region of the back panel of the skirt cover or trunk cover may be non-refastenably engaged to the front waist band region and the back waist band region or with the waist elastic members.

## Description

### Background of the Invention

This invention pertains to a process friendly disposable garment, and more particularly to a disposable garment that may be adapted to provide containment and absorbency of waste matter while being useful as active and swim wear. This invention pertains to a process for manufacturing garments, and more particularly to a cost effective, high speed method for manufacturing shorts or trousers.

Currently, disposable waste containment garments find widespread use in the areas of adult care, infant care, and child care, and have generally replaced reusable cloth garments. Disposable diapers, for example, have met a particular need and have become very popular. Disposable training pants have also met a particular need and have become popular. A problem exists with the design of active and swim wear. Neither active or swim wear is designed to accommodate a waste containment structure. Further, the design of the current active and swim wear does not keep waste containment structures in place during swimming and other activities.

Typically, the construction of trousers or shorts employs a multi-step process using multiple pieces of fabric, such as cloth or woven materials. The pieces of fabric are cut from a larger bolt of fabric into specified shapes. The pieces are sewn together, forming a finished garment. This process is labor and time intensive.

In the area of disposable garments, such as children bibs, coveralls, or examinations gowns, a more continuous process is used. Material, such as paper or plastic, is unwound from a roll. Strategically placed cuts are made in the material, forming head, arms, or legs openings. However, these disposable garments have several limitations, both in the design and durability necessary for active wear garments.

### Summary of the Invention

Thus, there is a need to provide an improved active and swim wear that minimizes the leakage of urine and fecal matter during a variety of activities including play, swimming, and travel to swimming while maintaining fecal containment during the activities. In response to this need, improved shorts and pant garments have been discovered.

Thus, there is a need to provide an improved process for manufacturing garments, including washable and disposable garments. There is also a need to provide comfortable and inexpensive active or swim wear garments. In addition, the garments need to be easy to put on and durable during wear. In response to this need, an improved cost effective, high speed process for manufacturing shorts and trousers has been discovered.

A waste containment garment according to the invention includes a waste containment structure having a longitudinal axis and opposite longitudinally spaced ends and a cover defining opposite waist regions. The waste containment structure comprises an absorbent core, a backsheet and an elastic member located in the waist regions to hold the structure in place. The elastic members are operatively joined to the cover. However, the waste containment structure can remain snugly in place while resisting movement in response to the cover.

The present invention relates to a disposable garment comprising a skirt cover having a front panel having two side edges and a waist region and an opposing bottom edge between the side edges and a back panel having two side edges and a waist region and an opposing bottom edge between the side edges. The garment also includes a pant structure having a front waist band region and a back waist band region longitudinally spaced and terminating in longitudinal ends, a crotch area between the front and back waist band regions, and a pair of side panels. The pant structure includes a waist opening, two leg openings and waist elastic members,
wherein the waist region of the front panel and the waist region of the back panel of the skirt cover are non-refastenably engaged to the front waist band region and the back waist band region having the waist elastic members positioned between the panels and the waist band regions.

In another aspect of the invention, a three-dimensional waste containment garment includes a waste containment structure and a cover. The full cover has an outer surface and an opposing inner surface and defines at least a waist opening. The cover is joined to the waste containment structure at least at a portion of the waist opening. A waste containment structure of the garment has a longitudinal axis, opposite longitudinally spaced ends, and side edges extending between the ends. The waste containment structure includes a liquid permeable liner, a backsheet attached to the liner, and an absorbent core sandwiched between the liner and backsheet. The cover is elastically connected to the ends of the waste containment structure.

One embodiment of the present invention is a continuous process for the manufacture of shorts or trousers to be worn about the lower body comprising an outer surface and an opposing inner surface, defining a waist opening and two leg openings. The present invention combines at least one web of fabric in a single continuous process to create shorts or trousers. Seaming can be accomplished by use of ultrasonics, heat sealing, adhesives, tape, or sewing, each offering a unique modification to the process.

The present invention relates to a continuous process for the manufacture of a shorts garment comprising:
a. providing four single layer webs of fabric including two side edges on each web of fabric;
b. aligning two of the four webs together in a side by side orientation;
c. bonding one side edge of each of the two webs in the side by side orientation together, defining at least a portion of an inseam and a first composite web;
d. aligning the remaining two of the four webs together in a side by side orientation;
e. bonding one side edge of each of the two webs in the side by side orientation together, defining at least another portion of the inseam and a second composite web;
f. aligning the first and second composite webs together in the face to face orientation, defining a arrangement having two layers of fabric and two top side edges and two bottom side edges;
g. intermittently bonding the composite webs wherein the bonding is accomplished in an alternating orientation near the center of the webs, defining center seams having a specific shape and an interior portion of fabric;
h. removing the interior portion of fabric, defining a cavity having a front to back contour to accommodate a human body;
i. folding one pair of the side edges together;
j. bonding the pair of side edges, defining at least one side seam and a tubular leg structure;
k. folding the other pair of side edges together;
l. bonding the other pair of side edges, defining at least another side seam and another tubular leg structure; and,
m. cutting the single web of fabric, defining discrete garment-sized pieces of fabric wherein each piece of fabric includes at least two side seams, an inseam, two tubular leg structures, and a waist opening.

Numerous features and advantages of the present invention will appear from the following description. In the description, reference is made to the accompanying drawings which illustrate desired embodiments of the invention. Such embodiments do not represent the full scope of the invention. Reference should, therefore, be made to the claims herein for interpreting the full scope of the invention.

### Brief Description of the Drawings

The above-mentioned and other features of the present invention and the manner of attaining them will become more apparent, and the invention itself will be better understood by reference to the following description of the invention, taken in conjunction with the accompanying drawings, wherein:
**Fig. 1** is a front view of a skirt cover and pant structure typifying an embodiment of the present invention.
**Fig. 2** is a front cut away view of a skirt cover and pant structure typifying an embodiment of the present invention.
**Fig. 3** is a cross sectional view of the waste containment structure.
**Fig. 4** is a front view of a trunk cover and pant structure typifying an embodiment of the present invention.
**Fig. 5** is a front cut away view of a trunk cover typifying an embodiment of the present invention.
**Fig. 6** is a cross-sectional view of the waste containment structure.
**Fig. 7** is a front cut-away view of a garment with a waste containment structure made by the present invention.
**Fig. 8** is a diagram of one embodiment of the present invention.

### Definitions

Within the context of this specification, each term or phrase below will include the following meaning or meanings:
(a) **"Bonded"** refers to the joining, adhering, connecting, attaching, or the like, of two elements. Two elements will be considered to be bonded together when they are bonded directly to one another or indirectly to one another, such as when each is directly bonded to intermediate elements.
(b) **"Bonded Carded Fabric or Web"** refers to fabric or webs made from staple fibers which are sent through a combing or carding unit, which breaks apart and aligns the staple fibers in the machine direction to form a generally machine direction-oriented fibrous nonwoven web. Such fibers are usually purchased in bales which are placed in a picker which separates the fibers prior to the carding unit. Once the web or fabric is formed, it is then bonded by one or more of several known bonding methods. Once such bonding method is powder bonding, wherein a powdered adhesive is distributed through the web or fabric and then activated, usually by heating the fabric and adhesive with hot air. Another suitable bonding method is pattern boding, wherein heated calendar rolls or ultrasonic bonding equipment are used to bond the fibers together, usually in a localized bond pattern, though the fabric can be bonded across its entire surface if so desired. Another suitable and well-known bonding method, particularly when using bi-component staple fibers, is through-air bonding.
(c) **"Cross Machine Direction"** means a direction generally perpendicular to the machine direction.
(d) **"Disposable"** includes being disposed of after use, and not intended to be washed and reused.
(e) **"Disposed", "disposed on", "disposed with", "disposed at", "disposed near"**, and variations thereof are intended to mean that one element can be integral or unitary with another element, or that one element can be a separate structure joined to or connected to or placed with or placed near another element.
(f) **"Elasticity" and "elastic"** include that property of a material by virtue of which it tends to substantially recover to its original size and shape after removal of a force causing deformation of the material.
(g) **"Elastically connected"** and **"elastically connecting"** refer to two elements being separated by and bonded to an elastic member, where the relative position of the two elements may change due to extension of the elastic member.
(h) **"Elongation"** includes the ratio of the extension of a material to the length of a material prior to the extension. Elongation is expressed in percent.
(i) **"Extension", "extend"**, and **"extended"** include the change in length of a material due to stretching. Extension is expressed in units of length.
(j) **"Fabric"** is used to refer to all of the woven, knitted, and nonwoven webs.
(k) **"Flexible"** refers to materials or fabrics that are compliant and readily conform to the general shape and contours of an individual's body.
(l) **"Force"** includes a physical influence exerted by one body on another which produces acceleration of bodies that are free to move and deformation of bodies that are not free to move. Force is expressed in grams-force.
(m) **"Foreshortened"** and **"foreshortening"** include to shorten beforehand, that is, before a subsequent step.
(n) **"Front"** and **"back"** are used to designate relationships relative to the garment itself, rather than to suggest any position the garment assumes when it is positioned on a wearer.
(o) **"Gatherable"** material is one which, when bonded to the reticular web with the latter under tension, will gather, with the formation of puckers or gathers, to accommodate contraction of the reticulated web upon release of the tensioning forces.
(p) **"Machine Direction"** means the direction in which it is produced or the length of fabric moving in the direction of the machine operations.
(q) **"Meltblown Fibers"** means fibers formed by extruding a molten thermoplastic material through a plurality of fine, usually circular, die capillaries as molten threads or filaments into converging high velocity, usually hot gas (e.g. air) streams which attenuate the filaments of molten thermoplastic material to reduce their diameter, which may be to microfiber diameter. Thereafter, the meltblown fibers are carried by the high velocity gas stream and are deposited on a collecting surface to form a web of randomly disbursed meltblown fibers. Such a process is disclosed, for example in U.S. Patent 3,849,241 to Butin, et al. Meltblown fibers are microfibers which may be continuous or discontinuous, are generally smaller than 10 microns in average diameter, and are generally tacky when deposited onto a collecting surface.
(r) **"Member"** when used in the singular can have the dual meaning of a single element or a plurality of elements.
s) **"Multi-layer Laminate"** means a laminate wherein some of the layers are spunbond and some are meltblown such as a spunbond/meltblown/spunbond (SMS) laminate and other as disclosed in U.S. Patent 4,041,203 to Brock et al., U.S. Patent 5,169,706 to Collier et al., U.S. Patent 5,145,727 to Potts et al., U.S. Patent 5,178,931 to Perkins, et al., and U.S. Patent 5,188,885 to Timmons et al. Such a laminate may be made by sequentially depositing onto a moving forming belt first a spunbond fabric layer, then a meltblown fabric layer and last another spunbond layer and then bonding the laminate in a manner described below. Alternatively, the fabric layers may be made individually, collected in rolls, and combined in a separate bonding step. Such fabrics usually have a basis weight of from about 0.1 to 12 osy (6 to 400 gsm), or more particularly from about 0.75 to about 3 osy. Multi-layer laminates may also have various numbers of meltblown layers or multiple spunbond layers in may different configurations and may include other materials like films or coform materials.
(t) **"Neckable Material"** means any material which can be necked.
(u) **"Necked Material"** refers to any material which has been constricted in at least one dimension by processes such as, for example, drawing or gathering.
(v) **"Non-elastic"** or **"Inelastic"** refers to any material that does not fall within the definition of "elastic".
(w) **"Nonwoven fabric or web"** means a web having a structure of individual fibers or threads which are interlaid, but not in an identifiable manner as in a knitted fabric. Nonwoven fabrics or webs have been formed from many processes such as, for example, meltblowing processes, spunbonding processes, and bonded carded web processes. The basis weight of nonwoven fabrics is usually expressed in ounces of material per square yard (osy) or grams per square meter (gsm) and the fiber diameters are usually expressed in microns.
(x) **"Operatively joined"** with reference to the attachment of an elastic member to another element means that the elastic member when attached to or connected to or treated with heat with the element gives that element elastic properties. With reference to the attachment of a non-elastic member to another element, it means that the member and element can be attached in any suitable manner that permits or allows them to perform the intended or described function of the joinder. The joining, attaching, connecting or the like can be either directly, such as joining either member directly to an element, or can be indirectly by means of another member or element disposed between the first member and the first element.
(y) **"Pattern"** includes any geometric or non-geometric form that can include, among others, a series of connected or unconnected lines or curves, a series of parallel or nonparallel or intersecting lines or curves, a series of linear or curvilinear lines, and the like, or any combinations thereof. The pattern can include a repeating form and/or non-repeating form.
(z) **"Pervious"** means that a layer of material is able to pass or transport a detectable amount of liquid under conditions normally encountered in a diaper/pant during use.

(aa) **"Porous"** means that a layer of material is able to pass or transport a measurable amount of liquid under conditions normally encountered in a diaper/pant during use.
(bb) **"Rupture"** includes the breaking or tearing apart of a material; in tensile testing, rupture refers to the total separation of a material into two parts either all at once or in stages, or the development of a hole in some materials.
(cc) **"Stretch bonded"** refers to an elastomeric strand being bonded to another member while the elastomeric strand is elongated at least about 25 percent of its relaxed length. Desirably, the term "stretch bonded" refers to the situation wherein the elastomeric strand is elongated at least about 100 percent, more desirably at least about 300 percent, of its relaxed length when it is bonded to the other member.
(dd) **"Stretch bonded laminate" ("SBL")** refers to a composite material having at least two layers in which one layer is a gatherable layer and the other layer is a stretchable, that is, elastic, layer. The layers are joined together when the stretchable layer is in a stretched condition so that upon relaxing the layers, the gatherable layer is gathered.
(ee) **"Spunbonded fibers"** refers to small diameter fibers which are formed by extruding molten thermoplastic material as filaments from a plurality of fine, usually circular capillaries or spinneret with the diameter of the extruded filaments then being rapidly reduced as by, for example, in U.S. Patent 4,340,563 to Appel et al., and U.S. Patent 3,692,618 to Dorschner et al., U.S. Patent 3, 802,817 to Matsuki et al., U.S. Patents 3,338,992 and 3,341,394 to Kinney, U.S. Patent 3,502,763 to Hartman, and U.S. Patent 3,542,615 to Dobo et al. Spunbond fibers are generally not tacky when they are deposited onto a collecting surface. Spunbond fibers are generally continuous and have average diameters (from a sample of at least 10) larger than 7 microns, more particularly, between about 10 and 20 microns.
(ff) **"Tension"** includes a uni-axial force tending to cause the extension of a body or the balancing force within that body resisting the extension.
(gg) **"Two-dimensional"** refers to a garment, such as a diaper, that can be opened and laid in a flat condition without destructively tearing any structure. This type of garment does not have continuous leg and waist openings when opened and laid flat, and requires a fastening device, such as adhesive tapes, to attach the garment about the wearer.
(hh) **"Three-dimensional"** refers to a finished garment similar to shorts or pants in that they have continuous leg and waist openings that are bounded by the material of which the garment is made. This type of garment can be opened and laid flat only by destructively tearing it. This type of garment may or may not have manually tearable seams.
(ii) **"Ultimate elongation"** includes the elongation at the point of rupture.

These definitions are not intended to be limiting and these terms may be defined with additional language in the remaining portion of the specification.

### Description of a Desired Embodiment

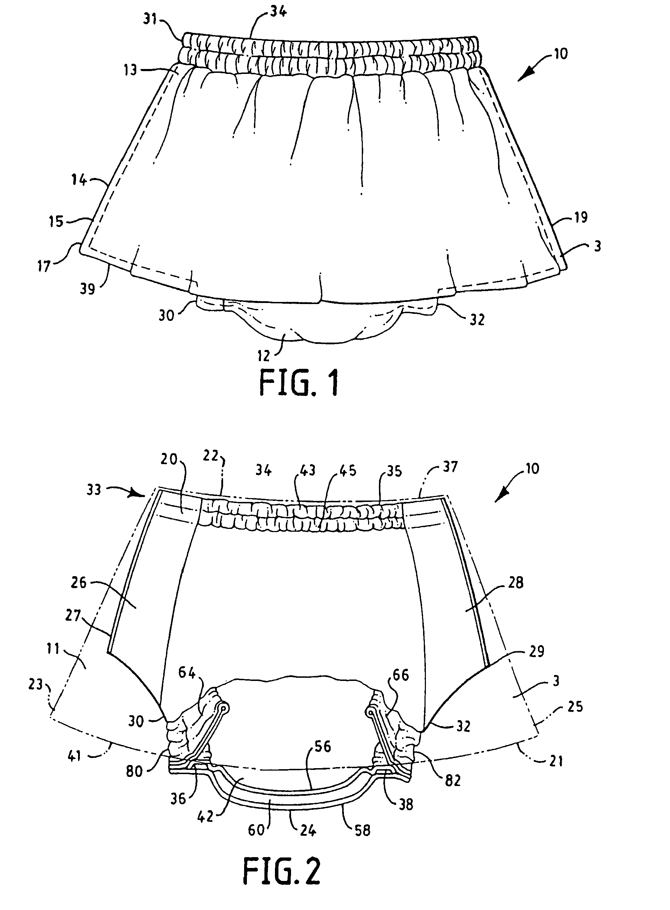

A garment **10,** a pant and skirt combination, of the present invention, as shown in **Figs. 1** and **2,** includes a pant structure **12** and a skirt cover **14.** The garment **10** is intended to resemble swimwear or outer active wear clothing. At the same time, the garment **10** may be constructed such that the skirt cover **14** remains securely in place about the wearer's waist with the pant structure **12** including a waste containment structure **42** positioned to receive and contain voided material. The garment **10** can be made or constructed in a variety of ways, one of which is described in U.S. Pat. Application Serial No. 043,132 filed on March 25, 1993, which is incorporated by reference herein. Other pant designs are described in U.S. Patents 4,938,757, 4,747,846, and 4,940,464; the contents of these three patents are incorporated by reference herein.

The skirt cover **14** includes opposing inner surface **11** and outer surface **13.** The skirt cover **14** is made up of a front panel **15** and a back panel **21.** The front panel **15** has a pair of side edges **17** and **19** and opposing waist region **31** and bottom **edge 39** positioned between the side edges **17** and **19.** The back panel **21** has a pair of side edges **23** and **25** and opposing waist region **33** and bottom edge **41** positioned between the side edges **23** and **25.** The front panel **15** and the back panel **21** may or may not extend to form an overlapping configuration at the sides of the garment 10.

In the desired embodiment of the present invention, the side edges **17, 19, 23,** and **25** are not joined or fastened together, forming a two-pieced skirt cover **14.** In another embodiment of the present invention, the side edge **17** is joined or fastened to the side edge **23** at the side seam **87** and the side edge **19** is joined or fastened to the side edge **25** at the side seam **89,** forming a one piece skirt cover **14.**

The side edges **17, 19, 23,** and **25** and the bottom edges **39** and **41** can be hemmed. For easier manufacture, the side edges **17, 19, 23,** and **25** and the bottom edges **39** and **41** are left unhemmed, facilitating easy machine cutoff.

In some embodiments of the present invention, the side seams **87** and **89** are non-refastenable. Non-refastenable seams **87** and **89** may be formed by any suitable means such as ultrasonic sealing, adhesive bonding, heat sealing, or the like. One suitable methods of forming such seams is disclosed in U.S. Patent 4,938,753 issued July 3, 1990, to Van Gompel et al., which is incorporated herein by reference. As illustrated most clearly in **Fig. 2,** the non-refastenable seams **27** and **29** of side panels **26** and **28** may be bonded together to form manually tearable, non-refastenable seams. The pant structure **12** thus defines a waist opening **34** and a pair of leg openings **30** and **32 (Figs. 1** and **2).**

In other embodiments of the present invention, the side seams **87** and **89** are refastenable. Refastenable means for securing the side edges **17, 19, 23,** and **25** include adhesives and mechanical type fasteners **96.** Mechanical type fasteners include buttons, button holes, snaps, buckles, clasps, hooks and loops, end extensions, tabs, and the like which are designed or adapted to interlock or engage some type of a complimentary device or the inner surface **11** or outer surface **13** of the skirt cover **14.** In addition, elasticized fasteners may also be used in assuring better fit of the garment **10.**

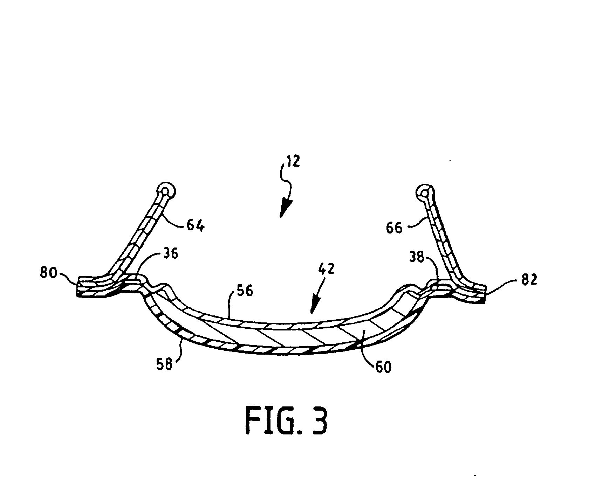

The pant structure **12** (See **Fig. 3)** includes a front and back longitudinally spaced waist band regions **20** and **22,** which terminate in longitudinal ends **35** and **37** of the pant structure **12.** A crotch area **24** is located between the front waist band region **20** and the back band waist region **22.** The left side panel **26** and the right side panel **28** extend between the front waist region **20** and the back waist region **22.** The pant structure **12** may include a waste containment section **42.** The waste containment structure **42** may include a backsheet **58,** a bodyside liner **56,** an absorbent core **60** as well as the side panels **26** and **28.** In some embodiments, containment flaps **64** and **66** are included in the waste containment structure **42.**

Side panels **26** and **28,** which may or may not have elastic elements, are ultrasonically bonded and are formed such that the materials of construction provide a manually tearable, non-refastenable region near the seams **27** and **29.** The side panels **26** and **28** can incorporate elastic elements which include incorporating a layer of elastic material or an SBL.

The pant structure **12** also desirably includes leg elastics **36** and **38** operatively joined to the crotch area **24.** The leg elastics **36** and **38** are positioned along the edges of side panels **26** and **28** and the longitudinal edges **80** and **82** of the pant structure **12** or the waste containment structure **42** in the crotch area **24.** The leg elastics **36** and **38** may assist in holding the pant structure **12,** and ultimately the waste containment structure **42** where present, against the body of the wearer or forming seals or gaskets about the legs of the wearer.

Leg elastics **36** and **38** can be stretch bonded to the cover material along the longitudinal edges of the pant structure **12.** The waist elastic **43** and **45** elasticizes the front and back waist band regions **20** and **22** of the pant structure **12.** Thereafter, each side panel **26** and **28** can be bonded together by seams **27** and **29** so that the pant structure **12** defines the waist opening **34** and the pair of leg openings **30** and **32.**

The pant structure **12** and the skirt cover **14** are joined at the waist of the garment **10.** The longitudinal ends **35** and **37** of the waist band regions **20** and **22** of the pant structure **12** are joined to the waist regions **31** and **33.** Waist elastic members **43** and **45** are positioned between the longitudinal ends **35** and **37** and the waist regions **31** and **33.**

The waist elastic members **43** and **45** may be stretch bonded to the waist regions **31** and **33** of the skirt cover **14** and the waist band regions **20** and **22** of the pant structure **12** or bonded in a relaxed state to a gathered portion of the waist band regions **20** and **22** of the pant structure **12** and the waist regions **31** and **33** of the skirt cover **14.** One suitable method for attaching the waist elastics **43** and **45** is disclosed in U.S. Patent 4,639,949 issued February 7, 1987, to Ales et al., which is incorporated herein by reference.

Desirably, the waist elastic members **43** and **45** are made up of at least two spunbond layers with elastic positioned between the spunbond layers. The longitudinal ends **35** and **37,** waist regions **31** and **33,** and the waist elastic members **43** and **45** are desirably bonded together by adhesives, however other methods of bonding discussed above can be utilized. The waist regions **31** and **33** of the skirt cover **14** may be attached to the pant structure **12** around the entirety of the waist opening **34** or only a portion thereof.

The longitudinal ends **35** and **37** and the waist regions **31** and **33** end at the top edge or near the top edge of the waist elastic members **43** and **45.** This allows the longitudinal ends **35** and **37** and the waist regions **31** and **33** to be cut off simultaneously.

The garment **10** may include a waste containment structure **42.** With reference to **Fig. 2,** the waste containment structure **42** as illustrated includes a backsheet **58,** a substantially liquid permeable bodyside liner **56,** and an absorbent core **60** sandwiched between the backsheet **58** and the bodyside liner **56.** The backsheet **58** and bodyside liner **56** are desirably longer and wider than the absorbent core **60,** so that the peripheries of the backsheet **58** and liner **56** form margins which may be sealed together using ultrasonic bonds, thermal bonds, adhesives, or other suitable means. The absorbent core **60** may be attached to the backsheet **58** and/or the bodyside liner **56** using ultrasonic bonds, adhesives, or other suitable means.

The waste containment structure **42** may also include additional components to assist in the acquisition, distribution and storage of waste material. For example, the waste containment structure **42** may include a transport layer, such as described in U.S. Patent 4,798,603 issued January 17, 1989, to Meyer et al., or a surge management layer, such as described in European Patent Application EP 0 539 703 A1, published May 5, 1993, which patent and application are incorporated herein by reference.

The waste containment structure **42** can be constructed by supplying bodyside liner and backsheet materials and sandwiching an individual absorbent core **60** between the backsheet **58** and bodyside liner **56.** The side peripheries of the backsheet **58** and bodyside liner **56** outward of the absorbent core **60** can be joined with side panel material and sealed together. Individual waste containment structure **42** can then be cut from the continuous supply of backsheet and bodyside liner materials. The waste containment structure **42** may optionally be T-shaped, I-shaped, hourglass-shaped, or irregularly-shaped.

The absorbent core **60** can comprise a coform material composed of a mixture of cellulosic fibers and synthetic polymer fibers. For example, the coform material may comprise an airlaid blend of cellulosic wood fibers and meltblown polyolefin fibers, such as polyethylene or polypropylene fibers. Absorbent core **60** can comprise only coform, or a combination of superabsorbent materials and coform, with other absorbent or non-absorbent materials.

The coform material may comprise an airlaid blend of cellulosic wood fibers and meltblown polyolefin fibers, such as polyethylene or polypropylene fibers, or may comprise an air-formed batt of cellulosic fibers (i.e., wood pulp fluff). Optionally, the absorbent core **60** may be treated with a surfactant to aid in liquid acquisition when in a dry environment. In particular embodiments of the invention, the absorbent core **60** has a bulk thickness of not more than about 1.25 cm when dry. The hydrophilic fibers and polymer strands may be provided in a fiber-to-polymer ratio which is less than 80:20, for example between about 30:70 and about 80:20 and, desirably between about 60:40 and about 70:30.

For absorbent core **60,** compounds to increase the core absorbency, are included in an effective amount and may consist of organic or inorganic high-absorbency materials. For example, the absorbent core **60** can include 0-5 weight percent high-absorbency material, desirably less than 1%. Suitable inorganic high-absorbency materials include, for example, absorbent clays and silica gels.

Organic high-absorbency materials can include natural materials, such as pectin, guar gum and peat moss, as well as synthetic materials, such as synthetic hydrogel polymers. Such hydrogel polymers may include, for example, carboxymethylcellulose, alkali metal salts of polyacrylic acids, polyacrylamides, polyvinyl alcohol, ethylene maleic anhydride copolymers, polyvinyl ethers, hydroxypropyl cellulose, polyvinyl morpholinone, polymers and copolymers of vinyl sulfonic acid, polyacrylates, polyacrylamides, polyvinyl pyridine or the like. Other suitable polymers can include hydrolyzed acrylonitrile grafted starch, acrylic acid grafted starch, and isobutylene maleic anhydride copolymers, and mixtures thereof.

The hydrogel polymers are desirably sufficiently cross-linked to render the materials substantially water-insoluble. Cross-linking may, for example, be by irradiation or by covalent, ionic, van der Waals or hydrogen bonding. Suitable materials are available from various commercial vendors, such as Dow Chemical Company, Hoechst-Celanese Corporation and Allied-Colloid. Typically, the high-absorbency material is capable of absorbing at least about 15 times its weight in water, and desirably is capable of absorbing more than about 25 times its weight in water.

The high-absorbency material can be distributed or otherwise incorporated into the absorbent core **60** employing various techniques. For example, the high-absorbency material can be substantially uniformly distributed among the fibers comprising the absorbent core **60.** The materials can also be non-uniformly distributed within the absorbent core **60** fibers to form a generally continuous gradient with either an increasing or decreasing concentration of high-absorbency material, as determined by observing the concentration moving inward from the backsheet **58.** Alternatively, the high-absorbency material can comprise a discrete layer separate from the fibrous material of the absorbent core **60,** or can comprise a discrete layer integral with the absorbent core 60.

The absorbent core **60** may also include a wrap layer (not shown) to help maintain the integrity of the fibrous core. This wrap may comprise a hydrophilic spunbond, meltblown or bonded-carded web material composed of synthetic polymer filaments, such as polypropylene, polyethylene, polyesters or the like or natural polymer filaments such as rayon or cotton.

The waste containment structure **42** most desirably incorporates a backsheet **58** which is vapor pervious and liquid pervious but only to a small degree liquid pervious. It is desirably associated with a cover structure (not shown) which is liquid impervious and which covers or separates the inner waste containment structure from contact with outside surfaces or people. The crotch area **24** of the waste containment structure **42** could be rendered liquid-impervious by appropriate means such as plastic film, while the upper portion and the waist opening **34** of the waste containment structure **42** could be covered by a liquid-pervious material, to aid in breathability.

The backsheet **58** may comprise a thin, liquid impermeable web or sheet of plastic film such as polyethylene, polypropylene, polyvinyl chloride or similar material. Alternately, the backsheet **58** may comprise a nonwoven, fibrous web which has been suitably constructed and arranged to have low liquid perviousness. Still alternately, the backsheet 58 may comprise a layered or laminated material, such as a thermally bonded plastic film and nonwoven web composite. Since the garment **10** is typically intended for active wear, an exposed backsheet or portions thereof, can be made of materials or of a basis weight which is abrasion resistant.

The backsheet **58** may be constructed of a single spunbonded polypropylene nonwoven web having a basis weight of about 0.5 oz/yd² (17 gsm) to about 2.0 oz/yd² (68 gsm). The skirt cover **14** desirably comprises a material having a basis weight of from about 0.5 oz/yd² (17 gsm) to about 2.0 oz/yd² (68 gsm), desirably 1.0 oz./yd² to 2.0 oz./yd² at least in the crotch and buttocks regions of the backsheet **58.** Lesser basis weights may be used in other regions of the garment **10.**

In the waste containment structure **42,** the backsheet **58** can also be liquid-pervious, and the cover liquid-impervious, for the same reasons as above. However, wherein the garment **10** has a skirt cover **14,** the crotch area **24** of the waste containment structure **42** could be rendered liquid-impervious by appropriate means such as a plastic film, while the upper portion of the waste containment structure **42** could be covered by a liquid-pervious material, to aid in breathability.

The bodyside liner **56** may be any soft, flexible, porous sheet which passes fluids therethrough. Again, the bodyside liner **56** must permit submersion in fresh water, salt water, or treated water and still retain its integrity. The bodyside liner 56 may comprise, for example, a nonwoven web or sheet of a spunbonded, meltblown or bonded-carded web composed of synthetic polymer filaments, such as polypropylene, polyethylene, polyesters or the like, or a web of natural polymer filaments such as rayon or cotton. The bodyside liner **56** has a pore size that readily allows the passage therethrough of liquids, such as urine and other body exudates. The bodyside liner **56** may be selectively embossed or perforated with discrete slits or holes extending therethrough. Suitable adhesives for adhering the laminate layers can be obtained from Findley Adhesives, inc. of Wauwatosa, Wisconsin.

As described previously, the side panels **26** and **28** may be formed of a material capable of stretching in one direction or capable of stretching in at least two substantially perpendicular directions. One suitable one-directional stretch material is disclosed in U.S. Patent 4,720,415 issued January 19, 1988, to Vander Wielen et al., which is incorporated herein by reference. The one-directional stretch material may comprise a composite material including at least one gatherable web bonded to at least one elongated elastic web.

The elastic web may be an elastic film or nonwoven fibrous elastic webs such as meltblown elastomeric fibrous webs. In one embodiment, the side panels **26** and **28** comprise a stretch bonded laminate formed of a pre-stretched elastic meltblown inner layer sandwiched between and attached to a pair of spunbond polypropylene nonwoven webs having a basis weight of about 0.4 oz/yd² (13.6 gsm). Suitable elastic materials can be purchased from the Shell Chemical Company of Houston, Texas under the trade name Kraton. Other suitable one-directional stretch materials are disclosed in U.S. Patents 4,606,964 issued August 19, 1986, to Wideman and 4,657,802 issued April 14, 1987, to Morman.

The material that can be used for the elastic element which can be used for the side panels **26** and **28** desirably has stretch characteristic in the first direction such that it is capable of from about 10 to about 500 percent elongation and upon release of tension will recover at least 55 percent of its elongation. It is generally desired that the material for use in the side panels **26** and **28** in the first direction be capable of between about 50 and about 300 percent elongation, particularly at least 125 percent elongation and recovery upon release of tension of at least 80 percent of its elongation.

Suitable two-directional stretch materials for the side panels **26** and **28** are disclosed in U.S. Patents 5,114,781 issued May 19, 1992, and 5,116,662 issued May 26, 1992, to Morman, which are incorporated herein by reference. A two-directional stretch material may comprise a composite material including a neckable material and an elastic sheet, which may be formed by meltblowing or extrusion. Neckable materials are those which may be constricted in at least one dimension by applying a tensioning force in a direction perpendicular to the desired direction of neck-down, and may include a spunbonded, meltblown or bonded carded web.

The tensioned, necked neckable material may be joined to the elongated elastic sheet at spaced locations arranged in a nonlinear configuration. Another two-directional stretch composite material may comprise one or more layers of reversibly necked material joined to one or more layers of elastic sheet at spaced locations. Reversibly necked materials are those that have been treated, such as with heat, while necked to impart memory to the material so that, when a force is applied to extend the material to its pre-necked dimensions, the treated, necked portions will generally recover to their necked dimensions upon termination of the force.

The leg elastics **36** and **38** and waist elastic members **43** and **45** may be formed of a stretch bonded laminate. In particular, the stretch bonded laminate may comprise at least one nonwoven gatherable layer and an elastic layer. Alternately, the leg elastics **36** and 38 and waist elastic **43** and **45** may be formed of a dry-spun coalesced multi-filament elastomeric thread sold under the tradename LYCRA and available from I.E. Du Pont de Nemours and Company. Still alternately, the leg elastics **36** and **38** and waist elastic members **43** and **45** may be formed of other typical elastics utilized in the diaper-making art, such as a thin ribbon of elastic material as disclosed in U.S. Patent 4,940,464 issued July 10, 1990, to Van Gompel et al., which is incorporated herein by reference. Elasticity could also be imparted to the longitudinal side sections by extruding a hot melt elastomeric adhesive between the backsheet **58** and the bodyside liner **56.** Other suitable elastic gathering means are disclosed in U.S. Patents 4,938,754 to Mesek and 4,388,075 to Mesek et al.

The skirt cover **14** can be desirably constructed of a single layer comprising film layer, nonwoven layer, or any other suitable liquid permeable or liquid impermeable material, desirably having a cloth-like feel. The skirt cover **14** is constructed of a single spunbonded polypropylene nonwoven web having a basis weight of about 0.5 oz/yd² (17 gsm) to about 2.0 oz/yd² (68 gsm).

The skirt cover **14** typically comprises a material having a basis weight of from about 0.5 oz/yd² (23.8 gsm) to about 2.0 oz/yd² (68 gsm). The skirt cover **14** may comprise a second layer of a liquid impermeable film layer suitably joined to the first layer by adhesive. The first layer of the skirt cover **14** may be spunbonded polypropylene nonwoven web having a basis weight of from about 0.5 oz/yd² (23.8 gsm) to about 2.0 oz/yd² (68 gsm). The second layer of the skirt cover **14** may be a polyethylene film ranging from about 0.5 to about 1.0 mil in thickness.

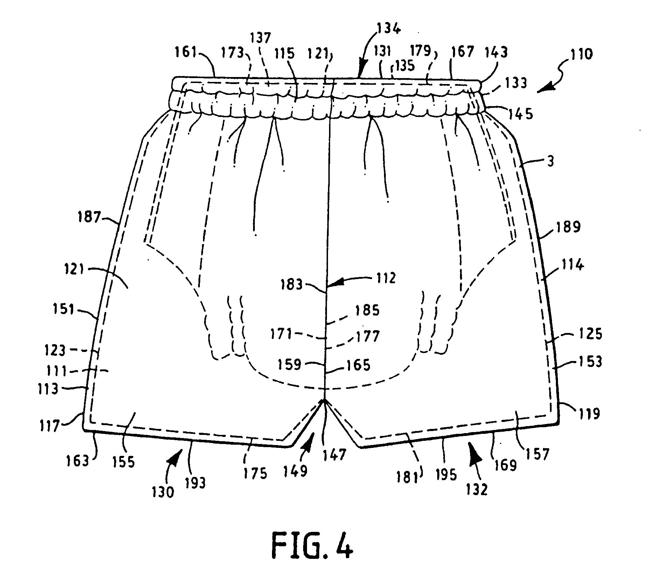

The shorts garment **110** (also referred to as trunk garment) is illustrated in **Fig. 4.** Within this application, the term "shorts" is understood to mean shorts, trousers, or any type of boxer styled garment having variable lengths of leg coverings. The trunk cover **114** includes opposing inner and outer surfaces **111** and **113.** According to the desired embodiment, the trunk cover **114** of the trunk garment **110** desirably comprises a right front panel **151,** a left front panel **153,** a right back panel **155,** and a left back panel **157.** The right front panel **151** has a pair of side edges **117** and **159** and opposing waist region **161** and bottom edge **163** positioned between the side edges **117** and **159.** The left front panel **153** has a pair of side edges **119** and **165** and opposing waist region **167** and bottom edge **169** positioned between the side edges **119** and **165.**

The right back panel **155** has a pair of side edges **123** and **171** and opposing waist region **173** and bottom edge **175** positioned between the side edges **123** and **171.** The left back panel **157** has a pair of side edges **125** and **177** and opposing waist region **179** and bottom edge **181** positioned between the side edges **125** and **177.**

The side edge **159** is joined to the side edge **165** at the center seam **183** forming a front waist region **131** and a front panel **115.** The side edge **171** is joined to the side edge **177** at the center seam **185** forming a back waist region **133** and a back panel **121.** The side edge **117** is joined to the side edge **123** at the side seam **187** and the side edge **119** is joined to the side edge **125** at the side seam **189.**

The front panel **115** and the back panel **121** of the trunk cover **114** are joined together at the inseam **147** so as to define a crotch section **149** extending centrally between the front and back panels **115** and **121** respectively. The front panel **115,** the back panel **121,** and the crotch section **149** when joined together define a waist opening **134,** and two leg openings **187** and **189** at opposite sides of the crotch section **149.**

In the embodiments of the trunk garment **110** where a pant structure **112** is not included, the waist regions **131** and **133** joined to waist elastic members **143** and **145** on the inner surface **111** of the trunk cover **114.** Although not as desirable, the waist elastic members **143** and **145** could be joined to the outer surface **113** of the trunk cover **114.**

The waist elastic members **143** and **145** may be stretch bonded to the waist regions **131** and **133** of the trunk cover **114** or bonded in a relaxed state to a gathered portion the waist regions **131** and **133** of the trunk cover **114.** One suitable method for attaching the waist elastics **143** and **145** is disclosed in U.S. Patent 4,639,949 issued February 7, 1987, to Ales et al., which is incorporated herein by reference.

Desirably, the waist elastic members **143** and **145** are made up of at least two spunbond layers with elastic positioned between the spunbond layers. The waist regions **131** and **133** and the waist elastic members **143** and **145** are desirably bonded together by adhesives, however other methods of bonding discussed above can be utilized. The waist regions **131** and **133** of the trunk cover **114** may be attached to the waist elastic members **143** and **145** around the entirety of the waist opening **134** or only a portion thereof.

The waist regions **131** and **133** end at the top edge or near the top edge of the waist elastic members **143** and **145.** This allows the waist regions **131** and **133** to be cut off simultaneously. The bottom edges **163, 169, 175,** and **181** can be hemmed. For easier manufacture, the bottom edges **163, 169, 175,** and **181** are left unhemmed, facilitating easy machine cutoff.

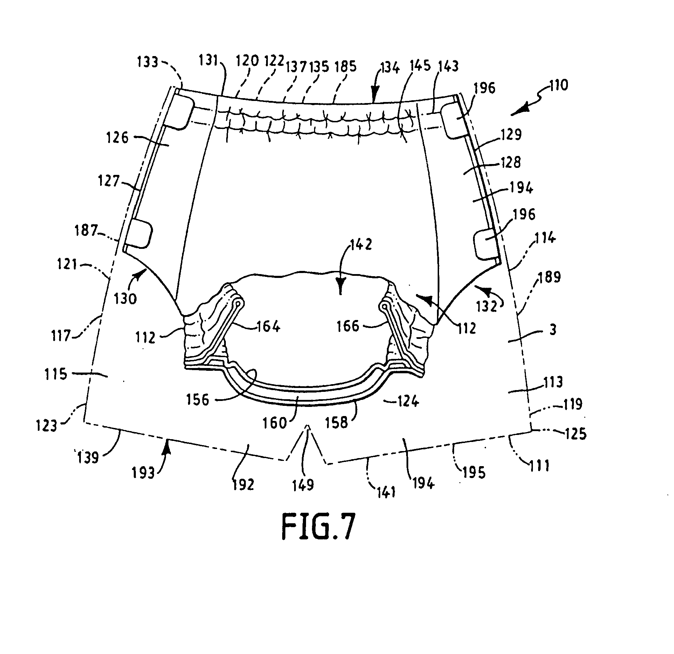

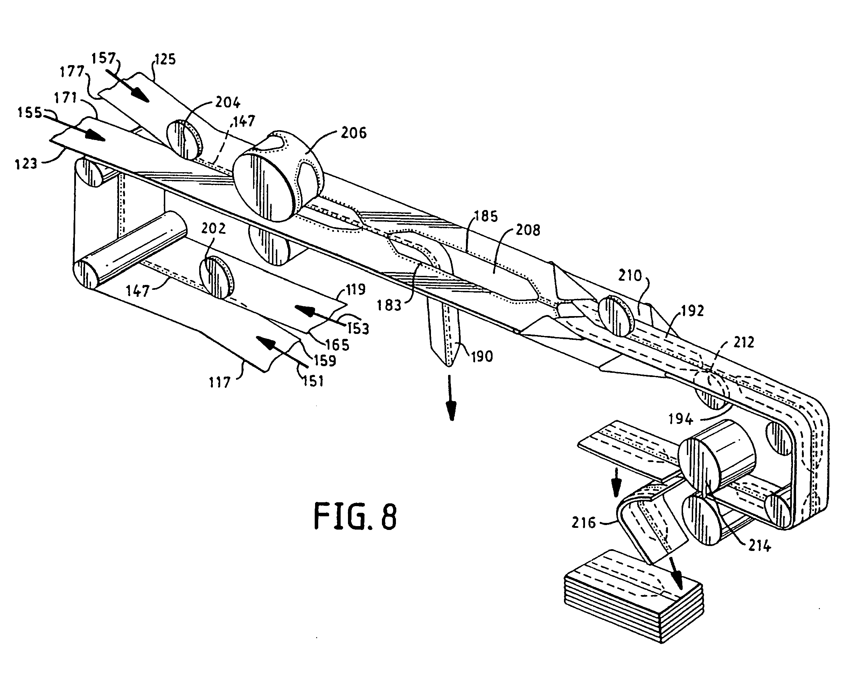

According to another embodiment of the present invention, see **Figs. 7** and **8,** the trunk cover **114** of the trunk garment **110** desirably comprises a front panel **115** and a back panel **121.** The front panel **115** has a pair of side edges **117** and **119** and opposing waist region **131** and bottom edge **139** positioned between the side edges **117** and **119.** The back panel **121** has a pair of side edges **123** and **125** and opposing waist region **133** and bottom edge **141** positioned between the side edges **123** and **125.** The side edge **117** is joined to the side edge **123** at the side seam **187** and the side edge 119 is joined to the side edge **125** at the side seam **189.**

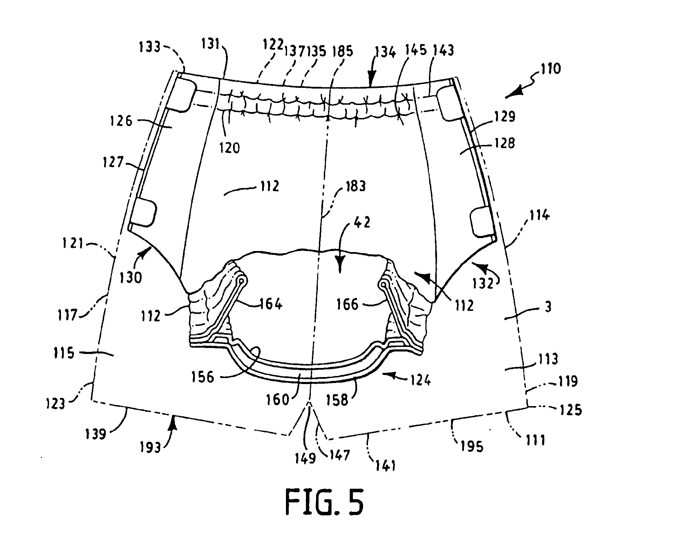

In some embodiments of the present invention of the garment **110,** the side seams **187** and **189,** the center seams **183** and **185,** and the seam **147** are non-refastenable. Non-refastenable seams **147, 183, 185, 187** and **189** may be formed by any suitable means such as ultrasonic sealing, adhesive bonding, heat sealing, or the like. One suitable methods of forming such seams is disclosed in U.S. Patent 4,938,753 issued July 3, 1990, to Van Gompel et al., which is incorporated herein by reference. As illustrated most clearly in **Fig. 5,** the non-refastenable seams **127** and **129** of side panels **126** and **128** may be bonded together to form manually tearable, non-refastenable seams. The pant structure **112** thus defines a waist opening **134** and a pair of leg openings **130** and **132 (Figs. 1** and **2).**

In other embodiments of the present invention of the garment **110,** the side seams **187** and **189,** the center seams **183** and **185,** and the inseam **147** are refastenable. Refastenable means for securing the edges including adhesives and mechanical type fasteners **96.** Mechanical type fasteners include buttons, button holes, snaps, buckles, clasps, hooks and loops, end extensions, tabs, and the like which are designed or adapted to interlock or engage some type of a complimentary device or the inner surface **111** or outer surface **113** of the trunk cover **114.** In addition, elasticized fasteners may also be used in assuring better fit of the garment **110.**

According to another embodiment of the garment **110,** see **Fig. 7,** the shorts **114** of the garment **110** desirably comprise a front panel **115** and a back panel **121.** The front panel **115** has a pair of side edges **117** and **119** and opposing waist region **131** and bottom edge **139** positioned between the side edges **117** and **119.** The back panel **121** has a pair of side edges **123** and **125** and opposing waist region **133** and bottom edge **141** positioned between the side edges **123** and **125.** The side edge **117** is joined to the side edge **123** at the side seam **187** and the side edge **119** is joined to the side edge **125** at the side seam **189.**

The front panel **115** and the back panel **121** of the trunk cover **114** are joined together at the inseam **147** so as to define a crotch section **149** extending centrally between the front and back panels **115** and **121** respectively. The front panel **115,** the back panel **121,** and the crotch section **149** when joined together define a waist opening **134,** and two leg openings **193** and **195** at opposite sides of the crotch section **149.**

In the embodiments of the trunk garment **110** where a pant structure **112** is not included, the waist regions **131** and **133** joined to waist elastic members **143** and **145** on the inner surface **111** of the trunk cover **114.** Although not as desirable, the waist elastic members **143** and **145** could be joined to the outer surface **113** of the trunk cover **114.**

The waist elastic members **143** and **145** may be stretch bonded to the waist regions **131** and **133** of the trunk cover **114** or bonded in a relaxed state to a gathered portion the waist regions **131** and **133** of the trunk cover **114.** One suitable method for attaching the waist elastics **143** and **145** is disclosed in U.S. Patent 4,639,949 issued February 7, 1987, to Ales et al., which is incorporated herein by reference.

Desirably, the waist elastic members **143** and **145** are made up of at least two spunbond layers with elastic positioned between the spunbond layers. The waist regions **131** and **133** and the waist elastic members **143** and **145** are desirably bonded together by adhesives, however other methods of bonding discussed above can be utilized. The waist regions **131** and **133** of the trunk cover **114** may be attached to the waist elastic members **143** and **145** around the entirety of the waist opening **134** or only a portion thereof.

The waist regions **131** and **133** end at the top edge or near the top edge of the waist elastic members **143** and **145.** This allows the waist regions **131** and **133** to be cut off simultaneously. The bottom edges **139** and **141** can be hemmed. For easier manufacture, the bottom edges **139** and **141** are left unhemmed, facilitating easy machine cutoff.

The trunk garment **110** can be formed in a continuous process by supplying a cover material including individual portions that define a single cover having waist regions **131** and **133** and front and back panels **115** and **121** extending from the waist regions **131** and **133.** The crotch section **149** is formed between the front and back panels **115** and **121.** The panels **115** and **121** can be shaped by die cutters, water jet cutters or other suitable means.

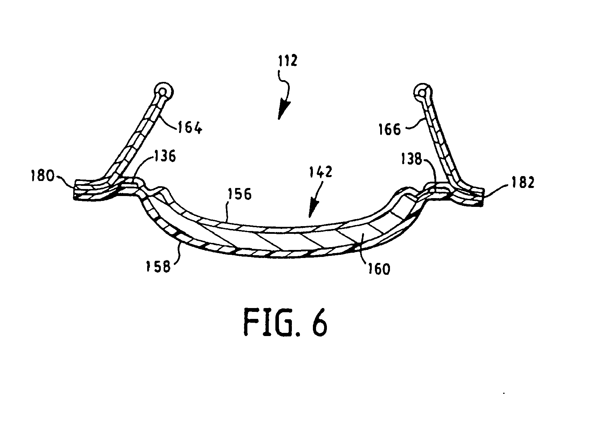

The pant structure **112** (See Fig. **5** and **6)** includes a front and back longitudinally spaced waist band regions **120** and **122,** which terminate in longitudinal ends **135** and **137** of the pant structure **112.** A crotch area **124** is located between the front waist band region **120** and the back band waist region **122.** The left side panel **126** and the right side panel **128** extend between the front waist region **120** and the back waist region **122.** The pant structure **112** may include a waste containment section **142.** The waste containment structure **142** may include a backsheet **158,** a bodyside liner **156,** an absorbent core **160** as well as the side panels **126** and **128.** In some embodiments, containment flaps **164** and **166** are included in the waist containment structure **142.**

Side panels **126** and **128,** which may or may not have elastic elements, are ultrasonically bonded and are formed such that the materials of construction provide a manually tearable, non-refastenable region near the seams **127** and **129.** The side panels **126** and **128** can incorporate elastic elements which include incorporating a layer of elastic material or an SBL.

The pant structure **112** also desirably includes leg elastics **136** and **138** operatively joined to the crotch area **124.** The leg elastics **136** and **138** are positioned along the edges of side panels **126** and **128** and the longitudinal edges **180** and **182** of the pant structure **112** in the crotch area **124.** The leg elastics **136** and **138** may assist in holding the pant structure **112,** and ultimately the waste containment structure **142** where present, against the body of the wearer or forming seals or gaskets about the legs of the wearer.

Leg elastics **136** and **138** can be stretch bonded to the cover material along the longitudinal edges of the pant structure **112.** The waist elastic **143** and **145** elasticizes the front and back waist band regions **120** and **122** of the pant structure **112.** Thereafter, each side panel **126** and **128** can be bonded together by seams **127** and **129** so that the pant structure **112** defines the waist opening **134** and the pair of leg openings **130** and **132.**

The pant structure **112** and the trunk cover **114** are joined at the waist of the trunk garment **110.** The longitudinal ends **135** and **137** of the waist band regions **120** and **122** of the pant structure **112** are joined to the waist regions **131** and **133.** Waist elastic members **143** and **145** are position between the longitudinal ends **135** and **137** and the waist regions **131** and **133.** The pant structure **112** is desirably attached to the front panel **115** and the back panel **121,** but not to the crotch section **149** of the cover **114.**

Desirably, the waist elastic members **143** and **145** are made up of at least two spunbond layers with elastic positioned between the spunbond layers. the longitudinal ends **135** and **137,** waist regions **131** and **133,** and the waist elastic members **143** and **145** are desirably bonded together by adhesives, however other methods of bonding discussed above can be utilized. The waist regions **131** and **133** of the trunk cover **114** may be attached to the pant structure **112** around the entirety of the waist opening **134** or only a portion thereof. The waist elastic members **143** and **145** may be stretch bonded to the cover **114** or bonded in a relaxed state to a gathered portion of the waist regions of the panels of the cover **114.** One suitable method was discussed above.

The waist elastic members **143** and **145** may be stretch bonded to the waist regions **120** and **122** of the trunk cover **114** and the waist band regions **120** and **122** of the pant structure **112** or bonded in a relaxed state to a gathered portion of the waist band regions **120** and **122** of the pant structure **112** and the waist regions **131** and **139** of the trunk cover **114.** One suitable method for attaching the waist elastics **143** and **145** is disclosed in U.S. Patent 4,639,949 issued February 7, 1987, to Ales et al., which is incorporated herein by reference.

The longitudinal ends **135** and **137** and the waist regions **131** and **133** end at the top edge or near the top edge of the waist elastic members **143** and **145.** This allows the longitudinal ends **135** and **137** and the waist regions **131** and **133** to be cut off simultaneously.

To construct the trunk cover **114** of the desired embodiment for trunk garment **110,** the front panel section **115** may be joined with the back panel **121** along seams **187** and **189** and at the inseam **147** in the crotch area **149** and to pant structure **112** at the front and the back waist band regions **120** and **122** near the waist opening **134.** The term "finished pant" means a three-dimensional pant that can be used for its intended purpose.

The trunk garment **110** may include a waste containment structure **142.** (See **Fig. 5)** The waste containment structure **142** as illustrated includes a backsheet **158,** a bodyside liner **156,** an absorbent core **160** sandwiched between the backsheet **158** and the bodyside liner **156.** The backsheet **158** and bodyside liner **156** are desirably longer and wider than the absorbent core **160,** so that the peripheries of the backsheet **158** and liner **156** form margins which may be sealed together using ultrasonic bonds, thermal bonds, adhesives, or other suitable means. The absorbent core **160** may be attached to the backsheet **158** and/or the bodyside liner **156** using ultrasonic bonds, adhesives, or other suitable means.

The waste containment structure **142** may also include additional components to assist in the acquisition, distribution and storage of waste material. For example, the waste containment structure **142** may include a transport layer, such as described in U.S. Patent 4,798,603 issued January 17, 1989, to Meyer et al., or a surge management layer, such as described in European Patent Application EP 0 538 703 A1, published May 5, 1993, which patent and application are incorporated herein by reference.

The waste containment structure **142** can be constructed by supplying bodyside liner and backsheet materials and sandwiching an individual absorbent core **160** between the backsheet **158** and bodyside liner **156.** The side peripheries of the backsheet **158** and bodyside liner **156** outward of the absorbent core **160** can be joined with side panel material and sealed together. Individual waste containment structure **142** can then be cut from the continuous supply of backsheet and bodyside liner materials. The waste containment structure **142** may optionally be T-shaped, I-shaped, hourglass-shaped, or irregularly-shaped.

The absorbent core **160** can comprise a coform material composed of a mixture of cellulosic fibers and synthetic polymer fibers. For example, the coform material may comprise an airlaid blend of cellulosic wood fibers and meltblown polyolefin fibers, such as polyethylene or polypropylene fibers. Absorbent core **160** can comprise only coform, or a combination of superabsorbent materials and coform, with other absorbent or non-absorbent materials.

The coform material may comprise an airlaid blend of cellulosic wood fibers and meltblown polyolefin fibers, such as polyethylene or polypropylene fibers, or may comprise an air-formed batt of cellulosic fibers (i.e., wood pulp fluff). Optionally, the absorbent core **160** may be treated with a surfactant to aid in liquid acquisition when in a dry environment. In particular embodiments of the invention, the absorbent core **160** has a bulk thickness of not more than about 1.25 cm when dry. The hydrophilic fibers and polymer strands may be provided in a fiber-to-polymer ratio which is less than 80:20, for example between about 30:70 and about 80:20 and, desirably between about 60:40 and about 70:30.

For absorbent core **160,** compounds to increase the core absorbency, are included in an effective amount and may consist of organic or inorganic high-absorbency materials. For example, the absorbent core **160** can include 0-5 weight percent high-absorbency material, desirably less than 1%. Suitable inorganic high-absorbency materials include, for example, absorbent clays and silica gels.

Organic high-absorbency materials can include natural materials, such as pectin, guar gum and peat moss, as well as synthetic materials, such as synthetic hydrogel polymers. Such hydrogel polymers may include, for example, carboxymethylcellulose, alkali metal salts of polyacrylic acids, polyacrylamides, polyvinyl alcohol, ethylene maleic anhydride copolymers, polyvinyl ethers, hydroxypropyl cellulose, polyvinyl morpholinone, polymers and copolymers of vinyl sulfonic acid, polyacrylates, polyacrylamides, polyvinyl pyridine or the like. Other suitable polymers can include hydrolyzed acrylonitrile grafted starch, acrylic acid grafted starch, and isobutylene maleic anhydride copolymers, and mixtures thereof.

The hydrogel polymers are desirably sufficiently cross-linked to render the materials substantially water-insoluble. Cross-linking may, for example, be by irradiation or by covalent, ionic, van der Waals or hydrogen bonding. Suitable materials are available from various commercial vendors, such as Dow Chemical Company, Hoechst-Celanese Corporation and Allied-Colloid. Typically, the high-absorbency material is capable of absorbing at least about 15 times its weight in water, and desirably is capable of absorbing more than about 25 times its weight in water.

The high-absorbency material can be distributed or otherwise incorporated into the absorbent core **160** employing various techniques. For example, the high-absorbency material can be substantially uniformly distributed among the fibers comprising the absorbent core **160.** The materials can also be non-uniformly distributed within the absorbent core **160** fibers to form a generally continuous gradient with either an increasing or decreasing concentration of high-absorbency material, as determined by observing the concentration moving inward from the backsheet **158.** Alternatively, the high-absorbency material can comprise a discrete layer separate from the fibrous material of the absorbent core **160,** or can comprise a discrete layer integral with the absorbent core **160.**

The absorbent core **160** may also include a wrap layer (not shown) to help maintain the integrity of the fibrous core. This wrap may comprise a hydrophilic spunbond, meltblown or bonded-carded web material composed of synthetic polymer filaments, such as polypropylene, polyethylene, polyesters or the like or natural polymer filaments such as rayon or cotton. It may also be a creped tissue paper comprised of wood pulp and a wet-strength resin, such as Kymene, a product of Hercules, Inc. of Wilmington, Delaware.

The waste containment structure **142** most desirably incorporates a backsheet **158** which is vapor pervious and liquid pervious but only to a small degree liquid pervious. It is desirably associated with a cover structure (not shown) which is liquid impervious and which covers or separates the inner waste containment structure from contact with outside surfaces or people. The crotch area **124** of the waste containment structure **142** could be rendered liquid-impervious by appropriate means such as plastic film, while the upper portion and the waist opening **134** of the waste containment structure **142** could be covered by a liquid-pervious material, to aid in breathability.

The backsheet **158** may comprise a thin, liquid impermeable web or sheet of plastic film such as polyethylene, polypropylene, polyvinyl chloride or similar material. Alternately, the backsheet **158** may comprise a nonwoven, fibrous web which has been suitably constructed and arranged to have low liquid perviousness. Still alternately, the backsheet **158** may comprise a layered or laminated material, such as a thermally bonded plastic film and nonwoven web composite. Since the garment **110** is typically intended for active wear, an exposed backsheet or portions thereof, can be made of materials or of a basis weight which is abrasion resistant.

The backsheet **158** may be constructed of a single spunbonded polypropylene nonwoven web having a basis weight of about 0.5 oz/yd² (17 gsm) to about 2.0 oz/yd² (68 gsm). The shorts **114** desirably comprises a material having a basis weight of from about 0.5 oz/yd² (17 gsm) to about 2.0 oz/yd² (68 gsm), desirably 1.0 oz./yd² to 2.0 oz./yd² at least in the crotch and buttocks regions of the backsheet **158.** Lesser basis weights may be used in other regions of the garment **110.**

In the waste containment structure **142,** the backsheet **158** can also be liquid-pervious, and the cover liquid-impervious, for the same reasons as above. However, wherein the garment **110** has shorts **114,** the crotch area **124** of the waste containment structure **142** could be rendered liquid-impervious by appropriate means such as a plastic film, while the upper portion of the waste containment structure **142** could be covered by a liquid-pervious material, to aid in breathability. In addition, however, the backsheet **158** of the waste containment structure **142** can be made with a vapor pervious material, to allow for some breathability of the structure, while the cover (not shown) is impervious, allowing for fast-drying and containment of any fluid passing through the structure.

The bodyside liner **156** may be any soft, flexible, porous sheet which passes fluids therethrough. Again, the bodyside liner **156** must permit submersion in fresh water, salt water, or treated water and still retain its integrity. The bodyside liner **156** may comprise, for example, a nonwoven web or sheet of a spunbonded, meltblown or bonded-carded web composed of synthetic polymer filaments, such as polypropylene, polyethylene, polyesters or the like, or a web of natural polymer filaments such as rayon or cotton. The bodyside liner **156** has a pore size that readily allows the passage therethrough of liquids, such as urine and other body exudates. The bodyside liner 156 may be selectively embossed or perforated with discrete slits or holes extending therethrough.

Optionally, the web or sheet may be treated with a surfactant to aid in liquid transfer. One suitable material for the bodyside liner **156** is a wettable spunbonded polypropylene web produced by the methods and apparatus described in U.S. Patents 4,340,563 issued July 20, 1982, and 4,405,297 issued September 23, 1983, to Appel et al., which are incorporated herein by reference. The bodyside liner 156 is liquid permeable and is a spunbonded polypropylene nonwoven web having a basis weight of about 0.75 oz/yd² (25.4 gsm). Suitable adhesives for adhering the laminate layers can be obtained from Findley Adhesives, Inc. of Wauwatosa, Wisconsin.

As described previously, the side panels **126** and **128** may be formed of a matenal capable of stretching in one direction or capable of stretching in at least two substantially perpendicular directions. One suitable one-directional stretch material is disclosed in U.S. Patent 4,720,415 issued January 19, 1988, to Vander Wielen et al., which is incorporated herein by reference. The one-directional stretch material may comprise a composite material including at least one gatherable web bonded to at least one elongated elastic web. The elastic web may be an elastic film or nonwoven fibrous elastic webs such as meltblown elastomeric fibrous webs.

The elastic web may be an elastic film or nonwoven fibrous elastic webs such as meltblown elastomeric fibrous webs. In one embodiment, the side panels **126** and **128** comprise a stretch bonded laminate formed of a pre-stretched elastic meltblown inner layer sandwiched between and attached to a pair of spunbond polypropylene nonwoven webs having a basis weight of about 0.4 oz/yd² (13.6 gsm). Suitable elastic materials can be purchased from the Shell Chemical Company of Houston, Texas under the trade name Kraton. Other suitable one-directional stretch materials are disclosed in U.S. Patents 4,606,964 issued August 19, 1986, to Wideman and 4,657,802 issued April 14, 1987, to Morman.

The material that can be used for the elastic element which can be used for the side panels **126** and **128** desirably has stretch characteristic in the first direction such that it is capable of from about 10 to about 500 percent elongation and upon release of tension will recover at least 55 percent of its elongation. It is generally desired that the material for use in the side panels **126** and **128** in the first direction be capable of between about 50 and about 300 percent elongation, particularly at least 125 percent elongation and recovery upon release of tension of at least 80 percent of its elongation.

Suitable two-directional stretch materials suitable for use in the side panels **126** and **128** are disclosed in U.S. Patents 5,114,781 issued May 19, 1992, and 5,116,662 issued May 26, 1992, to Morman, which are incorporated herein by reference. A two-directional stretch material may comprise a composite material including a neckable material and an elastic sheet, which may be formed by meltblowing or extrusion. Neckable materials are those which may be constricted in at least one dimension by applying a tensioning force in a direction perpendicular to the desired direction of neck-down, and may include a spunbonded, meltblown or bonded carded web.

The tensioned, necked neckable material may be joined to the elongated elastic sheet at spaced locations arranged in a nonlinear configuration. Another two-directional stretch composite material may comprise one or more layers of reversibly necked material joined to one or more layers of elastic sheet at spaced locations. Reversibly necked materials are those that have been treated, such as with heat, while necked to impart memory to the material so that, when a force is applied to extend the material to its pre-necked dimensions, the treated, necked portions will generally recover to their necked dimensions upon termination of the force.

The leg elastics **136** and **138** and waist elastic members **143** and **145** may be formed of a stretch bonded laminate. In particular, the stretch bonded laminate may comprise at least one nonwoven gatherable layer and an elastic layer. Alternately, the leg elastics **136** and **138** and waist elastic **143** and **145** may be formed of a dry-spun coalesced multi-filament elastomeric thread sold under the tradename LYCRA and available from I.E. Du Pont de Nemours and Company.

Still alternately, the leg elastics **136** and **138** and waist elastic members **143** and **145** may be formed of other typical elastics utilized in the diaper-making art, such as a thin ribbon of elastic material as disclosed in U.S. Patent 4,940,464 issued July 10, 1990, to Van Gompel et al., which is incorporated herein by reference. Elasticity could also be imparted to the longitudinal side sections by extruding a hot melt elastomeric adhesive between the backsheet **158** and the bodyside liner **156.** Other suitable elastic gathering means are disclosed in U.S. Patents 4,938,754 to Mesek and 4,388,075 to Mesek et al. The trunk cover **114** can be desirably constructed of a single layer comprising film layer, nonwoven layer, or any other suitable liquid permeable or liquid impermeable material, desirably having a cloth-like feel. The trunk cover **114** is constructed of a single spunbonded polypropylene nonwoven web having a basis weight of about 0.5 oz/yd² (17 gsm) to about 2.0 oz/yd² (68 gsm). In the case of trunk garment **110,** the trunk cover **114** desirably comprises a material having a basis weight of from about 0.5 oz/yd² (17 gsm) to about 2.0 oz/yd² (68 gsm), desirably 1.0 oz./yd² to 2.0 oz./yd² at least in the crotch and buttocks regions of the trunk cover **114.**

The shorts **114** may comprise a second layer of a liquid impermeable film layer suitably joined to the first layer by adhesive. The first layer of the shorts **114** may be spunbonded polypropylene nonwoven web having a basis weight of from about 0.5 oz/yd² (17 gsm) to about 2.0 oz/yd² (68 gsm). The second layer of the shorts **114** may be a polyethylene film ranging from about 0.5 to about 1.0 mil in thickness.

The present invention is a continuous process for the manufacture of shorts **114** (or trousers) to be worn about the lower body comprising an outer surface **113** and an opposing inner surface **111,** defining a waist opening **134** and two leg openings **193** and **195.** The present invention requires at least one web of fabric in a single continuous process to create shorts **114** or trousers. Seaming can be accomplished by use of ultrasonics, heat sealing, adhesives, tape, or sewing, each offering a unique modification to the process.

In one embodiment of the present invention (see Fig. 8), four panels **151, 153, 155,** and **157** (also referred to as webs) of sufficient width of fabric to make the garment **110** are combined to produce shorts **114.** The desired fabric is nonwoven although any disposable or washable fabric can be used. Two of the panels **151** and **153** of fabric are unwound from rolls and brought together side by side and bonded **202** together at one side edge of each of the panels **151** and **155,** defining one half of the inseam seam **147** on the composite web. The desired method of bonding is ultrasonics, although other methods of bonding, such as heat sealing, adhesives, tape, or sewing can be used. The other two panels **153** and **157** are treated **204** in an identical manner, creating a second composite web and the other half of the inseam **147.**

The two composite panels (also referred to as front and back panels) of **151** and **155;** and **153** and **157** are then brought together, face to face, resulting in an arrangement made up of two layers of fabric and having four side edges **117, 119, 123,** and **125** wherein two side edges **(117** and **119; 123** and **125)** are adjacent each other, one on top of the other, on each side of the bonding.

The two composite webs **(151** and **153, 155** and **157)** are then subjected to a crotch bonder **206** wherein a bond, defining center seams **183** and **185,** is applied in approximately the shape of an asymmetrical oval near the center of the fabric. (A variety of shapes may be used, symmetrical as well as asymmetrical.) The bonding is applied intermittently and with alternating orientation of top of oval to top of oval and bottom of oval to bottom of oval. (A repeating orientation of top of oval to bottom of oval may also be used.) The distance between the bonding defining the center seams **183** and **185** is varied depending on the desired length of leg covering for the garment. If trousers are desired, more distance is placed between the oval bonding, thereby providing more leg covering. If shorts are desired, less distance is placed between the oval bonding, thereby providing less leg covering.

The interior section **190** of the oval bonding, the portion inside of the center seams **183** and **185,** is cut **208,** desirably die cut or ultrasonically cut, defining a cavity having a front to back contour to accommodate a human body in the finished garment **110.** The interior section **190** can be removed by any method known in the art, desirably a vacuum source.

The top two side edges **117** and **123** are then folded back **210** to meet or overlap. The top side edges **117** and **123** are bonded, defining one side seam **127** and a tubular leg structure **192.** The bottom two side edges **119** and **125** are then folded back **212** to meet or overlap. The bottom side edges **119** and **125** are bonded, defining the other side seam **129** and another tubular leg structure **194.** If desired, the bottom side edges **119** and **125** could be folded and bonded together before the top side edges **117** and **123** were folded and bonded together. At this point in the process, the fabric has opposing tubular leg structures **192** and **194,** one on each side of the center seams **183** and **185,** side seams **127** and **129,** and the inseam **147.**

The fabric is then put through the final cut off knife **214** (or any other cutting device appropriate for this process), defining discrete garments **110** having waist regions **161, 167, 173,** and **179,** a waist opening **134,** and leg openings **193** and **195.** The discrete garments **110** are produced in alternating orientation of waist to waist and then leg to leg.

Every other garment **110** can be flipped **216** so that the orientation of all of the garments **110** match. In an alternate embodiment, every other garment **110** is transported to a second stack of garments **110.**

Each of the garments **110** is now ready for the addition of waist elastics **143** and **145,** pant structure **112,** waste containment structure **142,** or any other feature desired for inclusion in the garments **110.**

In another embodiment of the present invention, two webs, **151** and **153,** of sufficient width of fabric are combined to produce shorts **114.** The desired fabric is nonwoven although any disposable or washable fabric can be used. The two webs **151** and **153** of fabric are unwound from rolls and brought together face to face, resulting in an arrangement made up of two layers of fabric and having four side edges **117, 119, 123,** and **125** wherein two side edges **(117** and **119; 123** and **125)** are adjacent each other, one on top of the other, on each side of the bonding defining the inseam **147.** The desired method of bonding is ultrasonics, although other methods of bonding, such as heat sealing, adhesives, tape, or sewing can be used.

The fabric is then subjected to a crotch bonder wherein a bond, defining center seams **183** and **185,** is applied in approximately the shape of an asymmetrical oval near the center of the fabric. (A variety of shapes may be used, symmetrical as well as asymmetrical.) The bonding is applied intermittently and with alternating orientation of top of oval to top of oval and bottom of oval to bottom of oval. (A repeating orientation of top of oval to bottom of oval may also be used.) The distance between the bonding defining the center seams **183** and **185** is varied depending on the desired length of leg covering for the garment. If trousers are desired, more distance is placed between the oval bonding, thereby providing more leg covering. If shorts are desired, less distance is placed between the oval bonding, thereby providing less leg covering.

The interior section **190** of the oval bonding, the portion inside of the center seams **183** and **185,** is cut, desirably die cut or ultrasonically cut, defining a cavity having a front to back contour to accommodate a human body in the finished garment **110.** The interior section **190** can be removed by any method known in the art, desirably a vacuum source.

The top two side edges **117** and **123** are then folded back to meet or overlap. The top side edges **117** and **123** are bonded, defining one side seam **127** and a tubular leg structure **192.** The bottom two side edges **119** and **125** are then folded back to meet or overlap. The bottom side edges **119** and **125** are bonded, defining the other side seam **129** and another tubular leg structure **194.** If desired, the bottom side edges **119** and **125** could be folded and bonded together before the top side edges **117** and **123** were folded and bonded together. At this point in the process, the fabric has opposing tubular leg structures **192** and **194,** one on each side of the center seams **183** and **185,** side seams **127** and **129,** and the inseam **147.**

The fabric is then put through the final cut off knife, defining discrete garments **110** each having waist regions **115** and **121,** a waist opening **134,** and leg openings **193** and **195.** The discrete garments **110** are produced in alternating orientation of waist to waist and then leg to leg. Every other garment **110** can be flipped so that the orientation of all of the garments **110** match. In an alternate embodiment, every other garment **110** is transported to a second stack of garments **110.**

Each of the garments **110** is now ready for the addition of waist elastics **143** and **145,** pant structure **112,** waste containment structure **142,** or any other feature desired for inclusion in the garments **110.**

In still another embodiment of the present invention, one web of fabric having sufficient width is processed to produce shorts **114.** The desired fabric is nonwoven although any disposable or washable fabric can be used. The one web of fabric is unwound from the roll and slit, or otherwise cut, length-wise into two webs **151** and **153** of fabric. The two webs **151** and **153** are brought together in a face to face orientation, resulting in an arrangement made up of two layers of fabric and having four side edges **117, 119, 123,** and **125** wherein two side edges **(117** and **119; 123** and **125)** are adjacent each other, one on top of the other, on each side of the bonding defining the inseam **147.** The desired method of bonding is ultrasonics, although other methods of bonding, such as heat sealing, adhesives, tape, or sewing can be used.

The fabric is then subjected to a crotch bonder wherein a bond, defining the center seams **183** and **185,** is applied in approximately the shape of an asymmetrical oval near the center of the fabric. (A variety of shapes may be used, symmetrical as well as asymmetrical.) The bonding is applied intermittently and with alternating orientation of top of oval to top of oval and bottom of oval to bottom of oval. (A repeating orientation of top of oval to bottom of oval may also be used.) The distance between the bonding defining the center seams **183** and **185** is varied depending on the desired length of leg covering for the garment. If trousers are desired, more distance is placed between the oval bonding, thereby providing more leg covering. If shorts are desired, less distance is placed between the oval bonding, thereby providing less leg covering.

The interior section **190** of the oval bonding, the portion inside of the center seams **183** and **185,** is cut, desirably die cut or ultrasonically cut, defining a center seam having a front to back contour to accommodate a human body in the finished garment. The interior section can be removed by any method known in the art, desirably a vacuum source.

The top two side edges **117** and **123** are then folded back to meet or overlap. The top side edges **117** and **123** are bonded, defining one side seam **127** and a tubular leg structure **192.** The bottom two side edges **119** and **125** are then folded back to meet or overlap. The bottom side edges **119** and **125** are bonded, defining the other side seam **129** and another tubular leg structure **194.** If desired, the bottom side edges **119** and **125** could be folded and bonded together before the top side edges **117** and **123** were folded and bonded together. At this point in the process, the fabric has opposing tubular leg structures **192** and **194,** one on each side of the center seams **183** and **185,** side seams **127** and **129,** and the inseam **147.**

The fabric is then put through the final cut off knife, defining discrete garments **110** having waist regions **115** and **121,** a waist opening **134,** and leg openings **193** and **195.** The discrete garments **110** are produced in alternating orientation of waist to waist and then leg to leg. Every other garment **110** can be flipped so that the orientation of all of the garments **110** match. In an alternate embodiment, every other garment **110** is transported to a second stack of garments **110.**

Each of the garments is now ready for the addition of waist elastics, pant structure, waste containment structure, or any other feature desired for inclusion.

The present invention relates to a disposable garment comprising **10** a skirt cover **14** having a front panel **15** having two side edges **17** and **19,** and a waist region **31,** and an opposing bottom edge **39** between the side edges **17** and **19,** and a back panel **21** having two side edges **23** and **25** and a waist region **33** and an opposing bottom edge **41** between the side edges **23** and **25;** and, a pant structure **12** having a front waist band region **20** and a back waist band region **22** longitudinally spaced and terminating in longitudinal ends **35** and **37,** a crotch area **24** between the front and back waist band regions **20** and **22,** and a pair of side panels **26** and **28,** the pant structure **12** having a waist opening **34,** two leg openings **30** and **32,** and waist elastic members **43** and **45,** wherein the waist region **31** of the front panel **15** and the waist region **33** of the back panel **21** of the skirt cover **14** are non-refastenably engaged to the front waist band region **20** and the back waist band region **22** having the waist elastic members **43** and **45** positioned between the panels **15** and **21** and the waist band regions **20** and **22.**

The disposable garment **10** may further comprise a waste containment structure **42** having a backsheet **58,** a bodyside liner **56,** and an absorbent core **60.** The disposable garment **10** wherein the side panels **26** and **28** of the pant structure **12** include elastic elements. The bottom edges **39** and **41** are hemmed. The skirt cover **14** extends below the crotch area **24** of the pant structure **12.** The side edges **17** and **19** of the front panel **15** and the back panel **21** of the skirt cover **14** are joined together to form a one piece skirt cover **14.**

Another embodiment of the present invention relates to a disposable garment comprising a skirt cover **14** having a front panel **15** having two side edges **17** and **19** and a waist region **31** and an opposing bottom edge **39** between the side edges **17** and **19** and a back panel **21** having two side edges **23** and **25** and a waist region **33** and an opposing second bottom edge **41** between the side edges **23** and **25;** a pant structure **12** having a front waist band region **20** and a back waist band region **22** longitudinally spaced and terminating in longitudinal ends **35** and **37,** a crotch area **24** between the front and back waist band regions **20** and **22,** and a pair of side panels **26** and **28,** the pant structure **12** having a waist opening **34,** two leg openings **30** and **32** and waist elastic members **43** and **45;** and, a waste containment structure **42** having a backsheet **58,** a bodyside liner **56,** and an absorbent core **60,** wherein the waist region **31** of the front panel **15** and the waist region **33** of the back panel **21** of the skirt cover **14** are non-refastenably engaged to the front waist band region **20** and the back waist band region **22** having the waist elastic members **43** and **45** positioned between the panels **15** and **21** and the waist band regions **20** and **22.**

Another embodiment of the present invention relates to a disposable garment **110** comprising a trunk cover **114** having a front panel **115** having two side edges **117** and **119** and a waist region **131** and an opposing bottom edge **139** between the side edges **123** and **125** and a back panel **121** having two side edges **123** and **125** and a waist region **133** and an opposing bottom edge **141** between the side edges **123** and **125,** wherein the side edges **117** and **123/119** and **125** are joined to form side seams **187** and **189** and the front panel **115** and the back panel **121** joined together at an inseam **147,** defining a crotch section **149** extending centrally between the front and back panels **115** and **121** defining a waist opening **134** and two leg openings **193** and **195** at opposite sides of the crotch section **149;** and, waist elastic members **143** and **145,** wherein the waist region **131** of the front panel **115** and the waist region **133** of the back panel **121** of the trunk cover **114** are non-refastenably engaged with the waist elastic members **143** and **145.**

The present invention further comprising a pant structure **112** having a front waist band region **120** and a back waist band region **122** longitudinally spaced and terminating in longitudinal ends **135** and **137,** a crotch area **124** between the front and back waist band regions **120** and **122,** and a pair of side panels **126** and **128,** the pant structure **112** having a waist opening **134** and two leg openings **130** and **132.** The present invention may also include the pant structure non-refastenably engaged to the waist elastics **143** and **145.**

Another embodiment of the present invention relates to a disposable garment **110** comprising a trunk cover **114** having a right front panel **151** having two side edges **117** and **159** and a waist region **161** and an opposing bottom edge **163** between the side edges **117** and **159,** a left front panel **153** having two side edges **119** and **165** and a waist region **167** and an opposing bottom edge **169** between the side edges **119** and **165,** a right back panel **155** having two side edges **123** and **171** and a waist region **173** and an opposing bottom edge **175** between the side edges **123** and **171,** and a left back panel **157** having two side edges **125** and **177** and a waist region **179** and an opposing bottom edge **181** between the side edges **125** and **177** wherein the side edges **117** and **123/119** and **125/159** and **165/171** and **177** are joined to form side seams **187** and **189** and center seams **183** and **185,** and the front panels **151** and **153** and the back panels **155** and **157** joined together at an inseam **147,** defining a crotch section **149** extending centrally between the front and back panels **151, 153, 155,** and **157** defining a waist opening **134** and two leg openings **193** and **195** at opposite sides of the crotch section **149;** and, waist elastic members **143** and **145,** wherein the waist regions **161** and **167** of the front panels **151** and **153** and the waist regions **173** and **179** of the back panels **155** and **157** of the trunk cover **114** are non-refastenably engaged with the waist elastic members **143** and **145.**

The present invention also relates to a disposable garment **110** comprising a trunk cover **114** having a front panel **115** having two side edges **117** and **119** and a waist region **131** and an opposing bottom edge **139** between the side edges **117** and **119** and a back panel **121** having two side edges **123** and **125** and a waist region **133** and an opposing bottom edge **141** between the side edges **119** and **123,** wherein the side edges **117** and **123/119** and **125** are joined to form side seams **187** and **189** and the front panel **115** and the back panel **121** joined together at an inseam **147,** defining a crotch section **149** extending centrally between the front and back panels **115** and **121** defining a waist opening **184** and two leg openings **186** and **188** at opposite sides of the crotch section **149;** a pant structure **112** having a front waist band region **120** and a back waist band region **122** longitudinally spaced and terminating in longitudinal ends **135** and **137,** a crotch area **149** between the front and back waist band regions **120** and **122,** and a pair of side panels **126** and **128,** the pant structure defining a waist opening **134** and two leg openings **130** and **132;** and, waist elastic members **143** and **145,** wherein the waist region **131** of the front panel **115** and the waist region **133** of the back panel **121** of the trunk cover **114** are non-refastenably engaged to the front waist band region **120** and the back waist band region **122** having the waist elastic members **143** and **145** positioned between the panels **115** and **121** and the waist band regions **120** and **122.**

The present invention further comprising a waste containment structure **142** having a backsheet **158,** a bodyside liner **156,** and an absorbent core **160.** The side panels **126** and **128** of the pant structure **112** include elastic elements.

The present invention relates to a disposable garment **110** comprising a trunk cover **114** having a right front panel **153** having two side edges **117** and **159** and a waist region **161** and an opposing bottom edge **163** between the side edges **117** and **159,** a left front panel **153** having two side edges **119** and **165** and a waist region 167 and an opposing bottom edge **169** between the side edges **119** and **165,** a right back panel **155** having two side edges **123** and **171** and a waist region **173** and an opposing bottom edge **175** between the side edges **123** and **171,** and a left back panel **157** having two side edges **125** and **177** and a waist region **179** and an opposing bottom edge **181** between the side edges **123** and **171** wherein the side edges **117** and **123/119** and **125/159** and **165/171** and **177** are joined to form side seams **187** and **189** and center seams **183** and **185,** and the front panels **151** and **153** and the back panels **155** and **157** joined together at an inseam **147,** defining a crotch section **149** extending centrally between the front and back panels **151, 153, 155,** and **157** defining a waist opening **134** and two leg openings **193** and **195** at opposite sides of the crotch section **149;** a pant structure **112** having a front waist band region **120** and a back waist band region **122** longitudinally spaced and terminating in longitudinal ends **135** and **137,** a crotch area **124** between the front and back waist band regions **120** and **122,** and a pair of side panels **126** and **128,** the pant structure **112** defining a waist opening **134** and two leg openings **130** and **132;** and, waist elastic members **143** and **145,** wherein the waist regions **161** and **167** of the front panels **151** and **153** and the waist regions **173** and **179** of the back panels **155** and **157** of the trunk cover **114** are non-refastenably engaged to the front waist band region **120** and the back waist band region **122** having the waist elastic members **143** and **145** positioned between the panels **151, 153, 155,** and **157** and the waist band regions **120** and **122.**

Another embodiment of the present invention relates to a continuous process for the manufacture of a shorts garment comprising:
a. providing four single layer webs **151, 153, 155,** and **157** of fabric 3 including two side edges **(117, 119, 123, 125, 159, 165, 171,** and **177)** on each web of fabric 3;
b. aligning two of the four webs **(151, 153, 155,** and **157)** together in a side by side orientation;
c. bonding one side edge **159** and **165** of each of the two webs **151** and **153** in the side by side orientation together, defining at least a portion of an inseam **147** and a first composite web;
d. aligning the remaining two of the four webs **(151, 153, 155,** and **157)** together in a side by side orientation;
e. bonding one side edge **171** and **177** of each of the two webs **155** and **157** in the side by side orientation together, defining at least another portion of the inseam **147** and a second composite web;
f. aligning the first and second composite webs together in the face to face orientation, defining an arrangement having two layers of fabric and two top side edges **117** and **123** and two bottom side edges **119** and **125;**
g. intermittently bonding the composite webs wherein the bonding is accomplished in an alternating orientation near the center of the webs, defining center seams **183** and **185** having a specific shape and an interior portion of fabric **3;**
h. removing the interior portion of fabric **3,** defining a cavity having a front to back contour to accommodate a human body;
i. folding one pair of the side edges **117** and **123** together;
j. bonding the pair of side edges **117** and **123,** defining at least one side seam **127** and a tubular leg structure **192;**
k. folding the other pair of side edges **119** and **125** together;
l. bonding the other pair of side edges **119** and **125,** defining at least another side seam **129** and another tubular leg structure **194;** and,
m. cutting the fabric **3,** defining discrete garment-sized pieces of fabric **3** wherein each piece of fabric **3** includes at least two side seams **127** and **129,** an inseam **147,** two tubular leg structures **192** and **194,** and a waist opening **134.**

The continuous process may further comprise turning every other garment-sized piece of fabric **3** over thereby providing garment-sized pieces of fabric **3** having the same orientation before the garment-sized pieces of fabric **3** are stacked. In the altemative, every other garment-sized pieces of fabric **3** may be placed in a second stack.

Another embodiment of the present invention relates to a continuous process for the manufacture of a shorts garment comprising:
a. providing four multi-layer laminate webs **151, 153, 155,** and **157** of fabric 3 including two side edges **(117, 119, 123, 125, 159, 165, 171,** and **177)** on each web of fabric **3;**
b. aligning two of the four webs **(151, 153, 155,** and **157)** together in a side by side orientation;
c. bonding one side edge **159** and **165** of each of the two webs **151** and **153** in the side by side orientation together, defining at least a portion of an inseam **147** and a first composite web;
d. aligning the remaining two of the four webs **(151, 153, 155,** and **157)** together in a side by side orientation;
e. bonding one side edge **171** and **177** of each of the two webs **155** and **157** in the side by side orientation together, defining at least another portion of the inseam **147** and a second composite web;
f. aligning the first and second composite webs together in the face to face orientation, defining a arrangement having two layers of fabric and two top side edges **117** and **123** and two bottom side edges **119** and **125;**
g. intermittently bonding the composite webs wherein the bonding is accomplished in an alternating orientation near the center of the webs, defining center seams **183** and **185** having a specific shape and an interior portion of fabric **3;**
h. removing the interior portion of fabric **3,** defining a cavity having a front to back contour to accommodate a human body;
i. folding one pair of the side edges **117** and **123** together;
j. bonding the pair of side edges **117** and **123,** defining at least one side seam **127** and a tubular leg structure **192;**
k. folding the other pair of side edges **119** and **125** together;
l. bonding the other pair of side edges **119** and **125,** defining at least another side seam **129** and another tubular leg structure **194;** and,
m. cutting the fabric **3,** defining discrete garment-sized pieces of fabric **3** wherein each piece of fabric **3** includes at least two side seams **127** and **129,** an inseam **147,** two tubular leg structures **192** and **194,** and a waist opening **134.**

The present invention also relates to a continuous process for the manufacture of a shorts garment comprising:
a. providing two single layer webs **151** and **153** of fabric **3** including two side edges **(117, 119, 123,** and **125)** on each web **151** and **153** of fabric **3;**
b. aligning the two webs **151** and **153** together in a face to face orientation, defining an arrangement having two layers of fabric and one pair of top side edges **117** and **123** and one pair of bottom side edges **119** and **125;**
c. intermittently bonding the webs **151** and **153** wherein the bonding is accomplished in an alternating orientation near the center of the fabric **3,** defining center seams **183** and **185** having a specific shape and an interior portion of fabric **3;**
d. removing the interior portion of fabric **3,** defining a cavity having a front to back contour to accommodate a human body;
e. folding one pair of side edges **117** and **123** together;
f. bonding the pair of side edges **117** and **123,** defining at least one side seam **127** and a tubular leg structure **192;**
g. folding the other pair of side edges **119** and **125** together;
h. bonding the other pair side edges **119** and **125,** defining at least another side seam **129** and another tubular leg structure **194;** and,
i. cutting the fabric **3,** defining discrete garment-sized pieces of fabric **3** wherein each piece of fabric **3** includes at least two side seams **127** and **129,** an inseam **147,** two tubular leg structures **192** and **194,** and a waist opening **134.**

One embodiment of the present invention relates to a continuous process for the manufacture of a shorts garment comprising:
a. providing two multi-layer laminate webs **151** and **153** of fabric **3** including two side edges (**117, 119, 123,** and **125**) on each web **151** and **153** of fabric **3;**
b. aligning the two webs **151** and **153** together in a face to face orientation, defining an arrangement having two layers of fabric and one pair of top side edges **117** and **123** and one pair of bottom side edges **119** and **125**;
c. intermittently bonding the webs **151** and **153** wherein the bonding is accomplished in an alternating orientation near the center of the fabric **3,** defining center seams **183** and **185** having a specific shape and an interior portion of fabric **3;**
d. removing the interior portion of fabric **3,** defining a cavity having a front to back contour to accommodate a human body;
e. folding one pair of side edges **117** and **123** together;
f. bonding the pair of side edges **117** and **123,** defining at least one side seam **127** and a tubular leg structure **192;**
g. folding the other pair of side edges **119** and **125** together;
h. bonding the other pair side edges **119** and **125,** defining at least another side seam **129** and another tubular leg structure **194;** and,
i. cutting the fabric **3,** defining discrete garment-sized pieces of fabric **3** wherein each piece of fabric **3** includes at least two side seams **127** and **129,** an inseam **147,** two tubular leg structures **192** and **194,** and a waist opening **134.**

The present invention also relates to a continuous process for the manufacture of a shorts garment comprising:
a. providing one single layer web of fabric **3;**
b. slitting the web of fabric length-wise thereby forming two single layer webs **151** and **153** of fabric **3** including two side edges **(117, 119, 123, a**nd **125)** on each web **151** and **153** of fabric **3;**
c. aligning the two webs **151** and **153** together in a face to face orientation, defining an arrangement having two layers of fabric and one pair of top side edges **117** and **123** and one pair of bottom side edges **119** and **125;**
d. intermittently bonding the webs **151** and **153** wherein the bonding is accomplished in an alternating orientation near the center of the fabric **3,** defining center seams **183** and **185** having a specific shape and an interior portion of fabric **3;**
e. removing the interior portion of fabric **3,** defining a cavity having a front to back contour to accommodate a human body;
f. folding one pair of side edges **117** and **123** together;
g. bonding the pair of side edges **117** and **123,** defining at least one side seam **127** and a tubular leg structure **192;**
h. folding the other pair of side edges **119** and **125** together;
i. bonding the other pair side edges **119** and **125,** defining at least another side seam **129** and another tubular leg structure **194;** and,
j. cutting the fabric **3,** defining discrete garment-sized pieces of fabric **3** wherein each piece of fabric **3** includes at least two side seams **127** and **129,** an inseam **147,** two tubular leg structures **192** and **194,** and a waist opening **134.**

Another embodiment of the present invention relates to a continuous process for the manufacture of a shorts garment comprising:
a. providing one multi-layer laminate web of fabric;
b. slitting the web of fabric length-wise thereby forming two single layer webs **151** and **153** of fabric **3** including two side edges (**117, 119, 123,** and **125)** on each web **151** and **153** of fabric **3;**
c. aligning the two webs **151** and **153** together in a face to face orientation, defining an arrangement having two layers of fabric and one pair of top side edges **117** and **123** and one pair of bottom side edges **119** and **125;**
d. intermittently bonding the webs **151** and **153** wherein the bonding is accomplished in an alternating orientation near the center of the fabric **3,** defining center seams **183** and **185** having a specific shape and an interior portion of fabric **3;**
e. removing the interior portion of fabric **3,** defining a cavity having a front to back contour to accommodate a human body;
f. folding one pair of side edges **117** and **123** together;
g. bonding the pair of side edges **117** and **123,** defining at least one side seam **127** and a tubular leg structure **192;**
h. folding the other pair of side edges **119** and **125** together;
i. bonding the other pair side edges **119** and **125,** defining at least another side seam **129** and another tubular leg structure **194;** and,
j. cutting the fabric **3,** defining discrete garment-sized pieces of fabric **3** wherein each piece of fabric **3** includes at least two side seams **127** and **129,** an inseam **147,** two tubular leg structures **192** and **194,** and a waist opening **134.**

The foregoing detailed description has been for the purpose of illustration. Thus, a number of modifications and changes may be made without departing from the spirit and scope of the present invention. For instance, alternative or optional features described as part of one embodiment can be used to yield another embodiment. Additionally, only one rather than both ends of the waste containment structure can be elastically connected to the cover. Therefore, the invention should not be limited by the specific embodiments described, but only by the claims.

The materials of which the garment **10** and the trunk garment **110** are made can be any materials specifically desired by the user or manufacturer. Numerous examples of materials used in constructing the garment **10** and the trunk garment **110** are described in the aforementioned U.S. patents and patent applications incorporated by reference herein.

## Claims

1. A disposable garment comprising:
a. a skirt cover having a front panel having two side edges and a waist region and an opposing bottom edge between the side edges and a back panel having two side edges and a waist region and an opposing bottom edge between the side edges; and
b. a pant structure having a front waist band region and a back waist band region longitudinally spaced and terminating in longitudinal ends, a crotch area between the front and back waist band regions, and a pair of side panels wherein the side panels of the pant structure include elastic elements, the pant structure having a waist opening, two leg openings and waist elastic members,
wherein the waist region of the front panel and the waist region of the back panel of the skirt cover are non-refastenably engaged to the front waist band region and the back waist band region having the waist elastic members positioned between the panels and the waist band regions.

2. The disposable garment according to Claim 1, further comprising a waste containment structure having a backsheet, a bodyside liner, and an absorbent core.

3. The disposable garment according to Claim 1 or 2, wherein the bottom edges are hemmed.

4. The disposable garment according to Claim 1, 2 or 3, wherein the skirt cover extends below the crotch area of the pant structure.

5. The disposable garment according to any preceding claim, wherein the side edges of the front panel and the back panel of the skirt cover are joined together to form a one piece skirt cover.

6. A disposable garment comprising:
a. a skirt cover having a front panel having two side edges and a waist region and an opposing bottom edge between the side edges and a back panel having two side edges and a waist region and an opposing second bottom edge between the side edges;
b. a pant structure having a front waist band region and a back waist band region longitudinally spaced and terminating in longitudinal ends, a crotch area between the front and back waist band regions, and a pair of side panels wherein the side panels of the pant structure include elastic elements, the pant structure having a waist opening, two leg openings and waist elastic members; and,
c. a waste containment structure having a backsheet, a bodyside liner, and an absorbent core, wherein the waist region of the front panel and the waist region of the back panel of the skirt cover are non-refastenably engaged to the front waist band region and the back waist band region having the waist elastic members positioned between the panels and the waist band regions.

7. A disposable garment comprising:
a. a trunk cover having a front panel having two side edges and a waist region and an opposing bottom edge between the side edges and a back panel having two side edges and a waist region and an opposing bottom edge between the side edges, wherein the side edges are joined to form side seams and the front panel and the back panel are joined together at an inseam, defining a crotch section extending centrally between the front and back panels defining a waist opening and two leg openings at opposite sides of the crotch section;
b. a pant structure having a front waist band region and a back waist band region longitudinally spaced and terminating in longitudinal ends, a crotch area between the front and back waist band regions, and a pair of side panels wherein the side panels of the pant structure include elastic elements, the pant structure having a waist opening and two leg openings; and
c. waist elastic members,
wherein the waist region of the front panel and the waist region of the back panel of the trunk cover are non-refastenably engaged with the waist elastic members.

8. The disposable garment according to Claim 7, wherein the pant structure is non-refastenably engaged to the waist elastics.

9. A disposable garment comprising:
a. a trunk cover having a front panel having two side edges and a waist region and an opposing bottom edge between the side edges and a back panel having two side edges and a waist region and an opposing bottom edge between the side edges, wherein the side edges are joined to form side seams and the front panel and the back panel are joined together at an inseam, defining a crotch section extending centrally between the front and back panels defining a waist opening and two leg openings at opposite sides of the crotch section;
b. a pant structure having a front waist band region and a back waist band region longitudinally spaced and terminating in longitudinal ends, a crotch area between the front and back waist band regions, and a pair of side panels wherein the side panels of the pant structure include elastic elements, the pant structure defining a waist opening and two leg openings; and,
c. waist elastic members,
wherein the waist region of the front panel and the waist region of the back panel of the trunk cover are non-refastenably engaged to the front waist band region and the back waist band region having the waist elastic members positioned between the panels and the waist band regions.

10. The disposable garment according to Claim 9, further comprising a waste containment structure having a backsheet, a bodyside liner, and an absorbent core.

11. The disposable garment according to Claim 9 or 10, wherein the bottom edges are hemmed.

12. A disposable garment comprising:
a. a trunk cover having a right front panel having two side edges and a waist region and an opposing bottom edge between the side edges, a left front panel having two side edges and a waist region and an opposing bottom edge between the side edges, a right back panel having two side edges and a waist region and an opposing bottom edge between the side edges, and a left back panel having two side edges and a waist region and an opposing bottom edge between the side edges wherein the side edges are joined to form side seams and center seams, and the front panels and the back panels joined together at an inseam, defining a crotch section extending centrally between the front and back panels defining a waist opening and two leg openings at opposite sides of the crotch section;
b. a pant structure having a front waist band region and a back waist band region longitudinally spaced and terminating in longitudinal ends, a crotch area between the front and back waist band regions, and a pair of side panels wherein the side panels of the pant structure include elastic elements, the pant structure defining a waist opening and two leg openings; and
c. waist elastic members,
wherein the waist regions of the front panels and the waist regions of the back panels of the trunk cover are non-refastenably engaged to the front waist band region and the back waist band region having the waist elastic members positioned between the panels and the waist band regions.

13. The disposable garment according to Claim 12, further comprising a waste containment structure having a backsheet, a bodyside liner, and an absorbent core.

14. The disposable garment according to Claim 12 or 13, wherein the bottom edges are hemmed.
